# EUROPEAN PATENT SPECIFICATION

(11) **EP 3 875 051 B1**
(45) Date of publication and mention of the grant of the patent: **04.12.2024**
(21) Application number: 19878767.3
(22) Date of filing: 15.10.2019
(51) Int. Cl.: A61B 46/20, A61L 31/14, A61L 31/12, A61L 31/10, A61L 31/06, A61L 31/04, A61L 31/16

(54) **SURGICAL DRAPE**
CHIRURGISCHES TUCH
CHAMP OPÉRATOIRE

(30) Priority: 30.10.2018 JP 2018203383
(43) Date of publication of application: 08.09.2021
(73) Proprietor: DAINICHISEIKA COLOR & CHEMICALS MFG. CO., LTD., Chuo-ku Tokyo 103-8383 (JP); National University Corporation Tokyo University Of Agriculture and Technology, Fuchu-shi Tokyo 183-8538 (JP)
(72) Inventor: OGAWA Shingo, Tokyo 103-8383 (JP); ASO Yu, Tokyo 103-8383 (JP); NAMIKI Ryosuke, Fuchu-shi, Tokyo 183-8538 (JP); TANAKA Ryo, Fuchu-shi, Tokyo 183-8538 (JP); UEMURA Akiko, Obihiro-shi, Hokkaido 080-8555 (JP)
(74) Representative: Wächtershäuser & Hartz Patentanwaltspartnerschaft mbB
(86) International application number: PCT/JP2019/040422
(87) International publication number: WO 2020/090435

(56) References cited:
- EP-A1- 0 716 672
- EP-A1- 2 146 846
- EP-B1- 0 716 672
- EP-B1- 2 146 846
- WO-A1-2013/101884
- WO-A1-2020/129009
- JP-A- 2013 502 281
- JP-A- 2015 123 642
- JP-A- H09 502 213
- US-A1- 2014 261 458
- US-A1- 2016 095 756
- US-A1- 2018 228 574
- US-A1- 2018 289 439

## Description

### Technical Field

The present invention relates to a surgical drape to be used by being disposed on the skin at the time of surgery. A surgical drape on which the preamble of claim 1 is based, is known from EP-A1 0 716 672 A1.

### Background Art

In the past, a surgical drape has been used for protecting surroundings of an incised part from infection and the like when a surgical operation is performed on a living body, such as a human or an animal. The surgical drape which is used for such a purpose is a sheet-like (or film-like) medical tool usually having a film-like base material retaining the shape of the drape itself and a tacky layer formed on the surface of this base material. When surgery is performed, the surface (tacky surface) of the tacky layer of the surgical drape is pasted to the surface of the skin including a part to be incised, and the skin is then incised together with the base material and the tacky layer using a surgical tool, such as a scalpel. Thereby, scatter and adhesion of blood, a chemical liquid, and the like to the skin surrounding the incised part is prevented, making it possible to proceed with the surgery.

Various surgical drapes have been developed so far. For example, a surgical drape obtained by pasting a resin film, a tackiness agent layer, and release paper together, wherein a partially separable cutoff line is formed on the release paper (Patent Literature 1), and a surgical drape obtained by pasting a resin film, a tackiness agent layer, and release paper together, wherein the side edge parts of the resin film are bonded strongly to the release paper (Patent Literature 2), and the like have been proposed.

In addition, a surgical drape having moderate moisture permeability, the surgical drape provided with a non-self-adhesive adhesive layer composed of a polyurethane gel adhesive, has been proposed (Patent Literature 3). Furthermore, a surgical drape having improved handling properties and the like, wherein a flexible film, an adhesive layer, and a liner are laminated, and handles are formed on the film and the liner, respectively, has been proposed (Patent Literature 4).
EP-A1 2 146 846 discloses multi-layer assembly that includes a release liner, a first layer disposed on the release liner, the first layer including a pressure-sensitive adhesive composition that includes a silicone polymer that includes silicone polyurea block copolymers, polydiorganosiloxane polymers, or combinations thereof, a second layer disposed on the first layer, the second layer including a composition that includes an elastomer, the composition of the second layer being different from the pressure-sensitive adhesive composition of the first layer.
US-A 2018/228574 dislcoses an anatomical drape, for covering a treatment area of an anatomical part, the drape comprising an elastomeric material capable of conforming to the contours of the anatomical part and including a curing agent contained within internal channels distributed in the drape; and wherein activation of the curing agent, for example by a light source, causes hardening of the material to at least partially set the drape in a configuration conforming to the anatomical part.

### Citation List

### Patent Literature

Patent Literature 1: Japanese Utility Model Laid-Open No. 59-53020
Patent Literature 2: Japanese Utility Model Laid-Open No. 62-48311
Patent Literature 3: Japanese Translation of PCT International Application Publication No. 1989-501287
Patent Literature 4: Japanese Translation of PCT International Application Publication No. 2001-526567

### Summary of Invention

### Technical Problem

Conventional surgical drapes can be pasted in a satisfactory state to a certain extent on the skin including a part to be incised. However, if the skin to which a surgical drape is to be pasted is an uneven surface having a low smoothness or a surface having a high curvature, such as a hand, a leg, or the head, a place incised or punctured with a surgical tool and its surroundings have been likely to peel off from the skin in some cases. The skin of an animal (other than a human) in particular, even when shaved before surgery, is in an uneven state due to the base of the remaining hair, and therefore a part subjected to incision or the like and its surroundings are likely to peel off from the skin. Once a tacky surface of a surgical drape peels off from the skin, it becomes easy for scattered blood and the like to enter the peeled part, so that it becomes difficult to proceed with surgery safely in some cases.

The present invention has been made in view of such problems of conventional techniques, and an object of the present invention is to provide a surgical drape such that even when it is incised or punctured with a surgical tool in a state where it is pasted to an uneven surface having a low smoothness or to a part having a high curvature, such as a hand, a leg, or the head, a part subjected to incision or the like and its surroundings are unlikely to peel off, the surgical drape having excellent handling properties and safety.

### Solution to Problem

That is, according to the present invention, a surgical drape according to claim 1 and described below is provided.
[1] A surgical drape provided with a tacky layer to be incised or punctured with a surgical tool, the tacky layer having a tacky surface to be brought into contact with a region including a surgery site of a living body, wherein the surgical drape has a Young's modulus in the range of 0.1 to 5.0 MPa or less, and having a piercing strength in a thickness direction of 4.0 N or less.
[2] The surgical drape according to [1], further provided with a film-like base material disposed by lamination on a surface on an opposite side of the tacky surface of the tacky layer.

### Advantageous Effects of Invention

According to the present invention, a surgical drape such that even when it is incised or punctured with a surgical tool in a state where it is pasted to an uneven surface having a low smoothness or to a part having a high curvature, such as a hand, a leg, or the head, a part subjected to incision or the like and its surroundings are unlikely to peel off, the surgical drape having excellent handling properties and safety, can be provided.

### Brief Description of Drawings

[Figure 1] Figure 1 is a partially sectional view schematically showing one embodiment of a surgical drape of the present invention.
[Figure 2] Figure 2 is a graph showing results of Evaluation (1) of Peeling Resistance Using Skin Model.
[Figure 3] Figure 3 is a graph showing results of Evaluation of Peeling Resistance Using Rat.

### Description of Embodiments

Hereinafter, embodiments of the present invention will be described, but the present invention is not limited to the following embodiments. The present inventors have conducted studies on the factor that makes a part of a drape, subjected to incision or the like, and its surroundings likely to peel off from the skin when the skin is incised in a state where the drape is pasted thereto. As a result, the present inventors have found that: when incision is performed pressing a surgical tool, such as a scalpel, against a drape pasted to the skin, the shape of the rigid drape is retained in the original shape, and the skin, which is flexible as compared to the drape, bends in the direction of pressing the surgical tool; and thereby, a slight gap is likely to occur between the skin and the tacky surface of the drape, and the part subjected to the incision or the like and its surroundings peel off from the skin because the gap which had occurred becomes the starting point. It is to be noted that the gap between the skin and the tacky surface occurs more remarkably when the surface of the skin is an uneven surface having a low smoothness or a surface having a high curvature. As a result of further studies, the present inventors have found that by making the drape itself "flexible" and "easily stretchable", the gap is made unlikely to occur between the skin and the tacky surface even when incision is performed pressing a surgical tool against the drape, and have thereby completed the present invention.

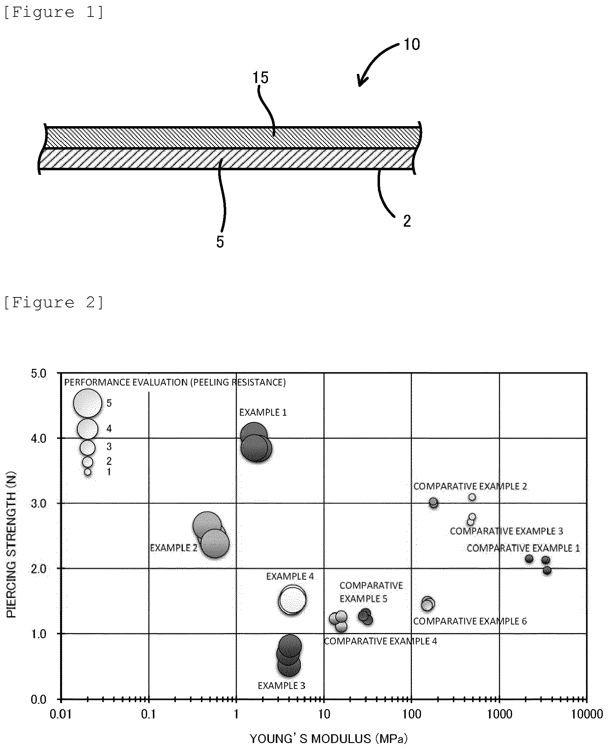

Figure 1 is a partially sectional view schematically showing one embodiment of the surgical drape (hereinafter, also simply referred to as "drape") of the present invention. As shown in Figure 1, a surgical drape 10 of the present embodiment is a sheet-like (film-like) medical tool provided with: a tacky layer 5 having a tacky surface 2; and a film-like base material 15 disposed by lamination on the surface on the opposite side of the tacky surface 2 of the tacky layer 5. The tacky surface 2 is a surface to be brought into contact with a region including a surgery site of a living body. The surgical drape 10 of the present embodiment has a Young's modulus of 10.0 MPa or less. That is, the surgical drape which is one embodiment of the present embodiment has a Young's modulus of a predetermined value or less and has properties, such as being more "flexible" and "easily stretchable". Thereby, in the surgical drape which is one embodiment of the present invention, the part subjected to incision or the like and its surrounding are unlikely to peel off even when it is incised or punctured with a surgical tool in a state where it is pasted to an uneven surface having a low smoothness or to a part having a high curvature, such as a hand, a leg, or the head.

When the Young's modulus exceeds 10.0 MPa, the flexibility and the stretchability are insufficient, and therefore the part subjected to incision or the like and its surroundings are likely to peel off from the skin. From the viewpoint of making the part subjected to incision or the like and its surroundings more unlikely to peel off, the surgical drape preferably has a Young's modulus of 10.0 MPa or less, more preferably 5.0 MPa or less, and particularly preferably 2.0 MPa or less. The lower limit value of the Young's modulus of the surgical drape is not particularly limited, but is preferably approximately 0.1 MPa or more from the viewpoint of an improvement in handling properties at the time of pasting before surgery and at the time of peeling after surgery, or the like.

The "Young's modulus" in the present specification is a physical property value which is measured by a tensile strength test in accordance with JIS K 6251:2010. It is to be noted that in the case of a drape having a laminated structure in which a base material and a tacky layer are laminated, the tacky layer is usually an extremely flexible part as compared to the base material and therefore the existence of the tacky layer hardly gives an influence on the Young's modulus of the whole drape. Therefore, in the case of the drape having a laminated structure in which a base material and a tacky layer are laminated, "the Young's modulus of the base material" can be regarded as being equal to "the Young's modulus of the drape".

The surgical drape may be such that it is not provided with a base material as long as it is provided with a tacky layer. When the surgical drape is not provided with a base material, the Young's modulus of the surgical drape means the Young's modulus of the tacky layer. It is to be noted that from the viewpoint of an improvement in handling properties at the time of pasting before surgery and at the time of peeling after surgery, or the like, the surgical drape 10 is preferably provided with the tacky layer 5, and the film-like base material 15 disposed by lamination on the surface on the opposite side of the tacky surface 2 of the tacky layer 5 as shown in Figure 1.

The tacky layer is composed of, for example, a tackiness agent similar to a tackiness agent constituting a tacky layer of a conventional surgical drape. Examples of the tackiness agent constituting the tacky layer include an acrylic-based tackiness agent, a silicone-based tackiness agent, a urethane-based tackiness agent, and a rubber-based tackiness agent. Any of various antibacterial ingredients, such as iodine and povidone iodine (iodine preparation), may be contained as necessary in the tacky layer.

The thickness of the tacky layer is not particularly limited and can appropriately be designed taking Young's modulus, tackiness strength, handling properties, and the like into consideration. The thickness of the tacky layer may be set to, for example, about 1 to about 1,200 µm, and may more preferably be 5 to 300 µm.

The base material is composed of, for example, a material similar to a material constituting a base material of a conventional surgical drape, such as a thermoplastic resin. Examples of the thermoplastic resin constituting the base material include: synthetic rubber such as nitrile rubber; natural rubber such as latex rubber; polyolefins such as polyurethanes, polyethylenes, and polypropylenes; and polyesters, polyvinyl chlorides, polystyrenes, polyvinyl acetates, polytetrafluoroethylenes, polysulfones, ABS resins, AS resins, acrylic resins, polyamides, polyacetals, polycarbonates, modified polyphenylene ethers, polyphenylene sulfides, polyether sulfones, amorphous polyarylates, liquid crystal polymers, polyether ether ketones, thermoplastic polyimides, and polyamide imides.

The thickness of the base material is not particularly limited and can appropriately be designed taking Young's modulus, handling properties, and the like into consideration. The thickness of the base material may be set to about 1 to about 200 µm, and may more preferably be 10 to 100 µm.

The surgical drape has a piercing strength in a thickness direction of 4.0 N or less, more preferably 3.5 N or less, and particularly preferably 3.0 N or less. Setting the piercing strength to 4.0 N or less can make the part subjected to incision or the like and its surroundings more unlikely to peel off even when the drape is incised or punctured with a surgical tool in a state where the drape is pasted to an uneven surface having a low smoothness or a part having a high curvature, such as a hand, a leg, or the head. It is to be noted that the lower limit value of the piercing strength of the surgical drape is not particularly limited, but is preferably approximately 0.5 N or more from the viewpoint of an improvement in handling properties at the time of pasting before surgery and at the time of peeling after surgery, or the like.

The "piercing strength" in the present specification is a physical property value which is measured by a piercing strength test in accordance with JIS Z 1707:1997. It is to be noted that in the case of a drape having a laminated structure in which a base material and a tacky layer are laminated, the tacky layer is usually an extremely flexible part as compared to the base material and therefore the existence of the tacky layer hardly gives an influence on the piercing strength of the whole drape. Therefore, in the case of the drape having a laminated structure in which a base material and a tacky layer are laminated, "the piercing strength of the base material" can be regarded as being equal to "the piercing strength of the drape".

Preferably, the surgical drape is further provided with release paper (liner) or the like disposed by lamination on the tacky surface of the tacky layer as necessary from the viewpoint of an improvement in handling properties, or the like. It is to be noted that the release paper is removed at the time of incision, and therefore the previously mentioned physical property values such as the "Young's modulus" and the "piercing strength" mean the physical property values of the drape excluding the release paper.

### Examples

Hereinafter, the present invention will specifically be described based on Examples, but the present invention is not limited to these Examples. It is to be noted that each of "parts" and "%" in Examples and Comparative Examples is on a mass basis unless otherwise noted.

### <Preparation of Base Materials>

Base materials 1 to 10 shown in Table 1 were prepared to measure the "Young's modulus" and the "piercing strength" for each base material. The results are shown in Table 1. It is to be noted that those described below were used as "Drape (1)", "Drape (2)", "Dressing material (1)", and "Dressing material (2).
· Drape (1): trade name "Ioban·Steri-Drape 2", Cat. #6035, manufactured by 3M Company, a polyester (including a tackiness agent layer composed of an acrylic-based tackiness agent, the tackiness agent layer having a thickness of 45 µm)
· Drape (2): trade name "Surgical Mate", manufactured by HOGY MEDICAL CO., LTD., a polyester (including a tackiness agent layer composed of an acrylic-based tackiness agent, the tackiness agent layer having a thickness of 30 µm)
· Dressing material (1): trade name "CATHEREEP FS ROLL", manufactured by NICHIBAN Co., Ltd., a polyurethane (including a tackiness agent layer composed of an acrylic-based tackiness agent, the tackiness agent layer having a thickness of 45 µm)
Dressing material (2): trade name "Tegaderm", manufactured by 3M Company, a polyurethane (including a tackiness agent layer composed of an acrylic-based tackiness agent, the tackiness agent layer having a thickness of 30 µm)

### (Young's Modulus)

The Young's modulus of each base material prepared was measured by a tensile strength test in accordance with JIS K 6251:2010. Specifically, each base material prepared was first punched using a JIS K 6251-3 dumbbell cutter to make 3 specimens (n=3). Subsequently, elongation (mm) and stress per section area (N/mm²), obtained by dividing stress (N) by the section area of each base material, were continuously measured using a force tester (trade name "STA-1150", manufactured by ORIENTEC CO., LTD.) while the specimen was being pulled at a rate of 100 mm/min to determine the initial slope indicating an elastic region and calculate the Young's modulus (MPa).

### (Piercing Strength)

The piercing strength of each base material prepared was measured by a piercing strength test in accordance with JIS Z 1707:1997. Specifically, a peak of the intensity at the time when a needle goes through each base material (n=3) was measured as the piercing strength (N).

**Table 1**

| | Material | Average thickness (µm) | Young's modulus (MPa) | | Piercing strength (N) | |
|---|---|---|---|---|---|---|
| | | | Individual | Average | Individual | Average |
| Base material 1 | Aluminum foil | 22 | 3,388 | 3,034 | 2.13 | 2.08 |
| | | | 2,188 | | 2.15 | |
| | | | 3,526 | | 1.97 | |
| Base material 2 | Polyvinilidene chloride | 10 | 180 | 178 | 3.02 | 3.01 |
| | | | 180 | | 2.99 | |
| | | | 176 | | 3.03 | |
| Base material 3 | Copy paper (cellulose) | 86 | 493 | 483 | 3.10 | 2.87 |
| | | | 467 | | 2.71 | |
| | | | 491 | | 2.79 | |
| Base material 4 | Nitrile rubber | 67 | 1.59 | 1.67 | 4.04 | 3.91 |
| | | | 1.79 | | 3.84 | |
| | | | 1.61 | | 3.85 | |
| Base material 5 | Latex rubber | 130 | 0.53 | 0.52 | 2.49 | 2.51 |
| | | | 0.46 | | 2.65 | |
| | | | 0.57 | | 2.38 | |
| Base material 6 | Drape (1) Polyester | 30 | 15.7 | 14.9 | 1.11 | 1.20 |
| | | | 13.3 | | 1.23 | |
| | | | 15.7 | | 1.26 | |
| Base material 7 | Drape (2) Polyester | 40 | 30.0 | 29.9 | 1.31 | 1.26 |
| | | | 31.7 | | 1.21 | |
| | | | 28.0 | | 1.27 | |
| Base material 8 | Dressing material (1) Polyurethane | 45 | 3.98 | 3.99 | 0.52 | 0.67 |
| | | | 3.88 | | 0.69 | |
| | | | 4.12 | | 0.81 | |
| Base material 9 | Polyethylene | 25 | 152 | 154 | 1.49 | 1.46 |
| | | | 159 | | 1.46 | |
| | | | 151 | | 1.43 | |
| Base material 10 | Dressing material (2) Polyurethane | 30 | 4.45 | 4.35 | 1.55 | 1.52 |
| | | | 4.23 | | 1.49 | |
| | | | 4.39 | | 1.51 | |

### <Production (1) of Drapes>

### (Example 1)

An acrylic-based tackiness agent was applied on silicone-coated paper in a thickness of 330 µm and then dried overnight to form a tacky layer having a thickness of about 100 µm. The formed tacky layer was pasted on one surface of base material 4 to obtain a drape (Example 1) having silicone-coated paper as release paper on a tacky surface. It is to be noted that the obtained drape was cut into an appropriate size in advance, and was used after releasing the silicone-coated paper when the performance was evaluated.

### (Examples 2 to 4 and Comparative Examples 1 to 6)

Drapes (Examples 2 to 4 and Comparative Examples 1 to 6) were obtained in the same manner as in Example 1 mentioned previously, except that the respective base materials shown in Table 2 were each used in place of base material 4. It is to be noted that with respect to the base materials (base materials 6 to 8 and 10) each having a tackiness agent layer as a part of the base material, the tacky layer was pasted on the surface of the tackiness agent layer to produce each drape.

### <Evaluation (1) of Peeling Resistance Using Skin Model >

The tacky surface of each produced drape was pasted to a skin model (trade name "Professional Skin Pad Mk2", manufactured by Limbs & Things) for practicing surgery, and the skin model was then bent into a columnar shape in such a way that the drape was on the outside to form a curved surface. A surgical scalpel (trade name "KAI No. 22", Cat.#522-B, manufactured by Kai Industries Co., Ltd.) was used to make a cut with a length of 3 to 4 cm into the drape on the skin model in a thickness (6 to 8 mm) that reached a subcutaneous layer (yellow sponge part). Three cuts were made for each drape in the same procedure (n=3). The degree of peeling-off of the tacky layer from the surface of the skin model at each part where the cut was made was observed visually to evaluate the peeling resistance according to the evaluation criteria described below. The results are shown in Table 2 and Figure 2 (graph).
5: The tacky layer was hardly peeled.
4: The tacky layer was peeled a little.
3: The tacky layer was peeled to an intermediate degree.
2: The tacky layer was peeled for the most part.
1: The tacky layer was peeled completely.

**Table 2**

| | Base material | Tacky layer | | Performance evaluation (Peeling resistance) | |
|---|---|---|---|---|---|
| | | Material | Thickness (µm) | Individual | Average |
| Comparative Example 1 | 1 | Acrylic-based tackiness agent | 100 | 2 | 1.3 |
| | | | | 1 | |
| | | | | 1 | |
| Comparative Example 2 | 2 | Acrylic-based tackiness agent | 100 | 2 | 1.3 |
| | | | | 1 | |
| | | | | 1 | |
| Comparative Example 3 | 3 | Acrylic-based tackiness agent | 100 | 1 | 1 |
| | | | | 1 | |
| | | | | 1 | |
| Example 1 | 4 | Acrylic-based tackiness agent | 100 | 5 | 4.7 |
| | | | | 5 | |
| | | | | 4 | |
| Example 2 | 5 | Acrylic-based tackiness agent | 100 | 5 | 5 |
| | | | | 5 | |
| | | | | 5 | |
| Comparative Example 4 | 6 | Acrylic-based tackiness agent | 100 | 2 | 2.3 |
| | | | | 2 | |
| | | | | 3 | |
| Comparative Example 5 | 7 | Acrylic-based tackiness agent | 100 | 2 | 1.7 |
| | | | | 1 | |
| | | | | 2 | |
| Example 3 | 8 | Acrylic-based tackiness agent | 100 | 5 | 4.3 |
| | | | | 5 | |
| | | | | 3 | |
| Comparative Example 6 | 9 | Acrylic-based tackiness agent | 100 | 2 | 2 |
| | | | | 2 | |
| | | | | 2 | |
| Example 4 | 10 | Acrylic-based tackiness agent | 100 | 5 | 4.6 |
| | | | | 4 | |
| | | | | 5 | |

### <Production (2) of Drapes>

### (Example 5)

A drape (Example 5) was obtained in the same manner as in Example 1 mentioned previously, except that base material 8 was used in place of base material 4 and that the thickness of the tacky layer formed using the acrylic-based tackiness agent was changed to 50 µm.

### (Comparative Example 7)

A drape (Comparative Example 7) was obtained in the same manner as in Example 1 mentioned previously, except that base material 6 was used in place of base material 4 and that the thickness of the tacky layer formed using the acrylic-based tackiness agent was changed to 45 µm.

### <Evaluation of Peeling Resistance Using Rat>

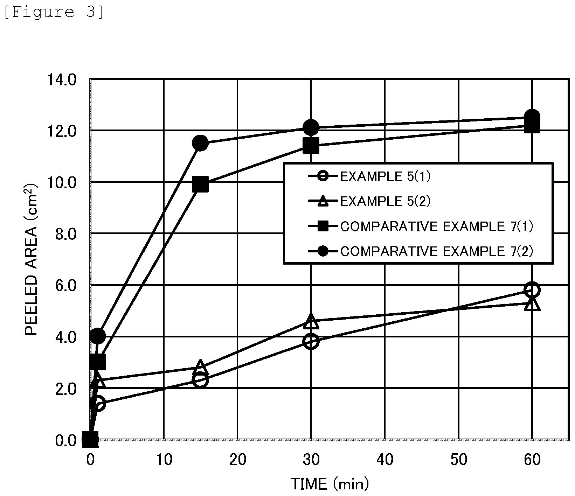

Hair of an SD rat (aged 3 to 4 months, without distinction of sex) was clipped with a pair of hair clippers, and the SD rat was then washed with a scrubbing agent. The SD rat was sterilized with chlorhexidine/alcohol and was then dried. The tacky surface of the drape cut into a size of 6 cm × 3 cm was pasted to the dorsal skin of the chest of the rat. After 30 minutes elapsed from pasting the tacky surface, the skin was cut from the base material side of the drape to a depth that reached subcutaneous tissue to make a cut with a length of 4 cm. India ink was dropped onto a cut skin part after a predetermined time to visualize a peeled part. Specifically, the cut skin part where the India ink had been dropped was photographed together with a scale, and the image was analyzed using image analysis software (Image-J), thereby calculating the peeled area. The results are shown in Table 3 and Figure 3 (graph).

**Table 3**

| | Base material | Tacky layer | | Peeled area (cm²) | | | | |
|---|---|---|---|---|---|---|---|---|
| | | Material | Thickness (µm) | Time (min) | | | | |
| | | | | 0 | 1 | 15 | 30 | 60 |
| Example 5 | 8 | Acrylic-based tackiness agent | 45 | 0.0 | 1.4 | 2.3 | 3.8 | 5.8 |
| | | | | 0.0 | 2.3 | 2.8 | 4.6 | 5.3 |
| Comparative Example 7 | 6 | Acrylic-based tackiness agent | 45 | 0.0 | 3.0 | 9.9 | 11.4 | 12.2 |
| | | | | 0.0 | 4.0 | 11.5 | 12.1 | 12.5 |

### <Production (3) of Drapes>

### (Example 6)

A drape (Example 6) was obtained in the same manner as in Example 1 mentioned previously, except that base material 8 was used in place of base material 4 and that the thickness of the tacky layer formed using the acrylic-based tackiness agent was changed to 50 µm (thickness of tacky layer (in total) : 95 µm).

### (Example 7)

A drape (Example 7) was obtained in the same manner as in Example 1 mentioned previously, except that base material 8 was used in place of base material 4 and that a tacky layer having a thickness of 50 µm (thickness of tacky layer (in total): 95 µm) was formed using a silicone-based tackiness agent.

### (Comparative Examples 8 and 9)

Drapes (Comparative Examples 8 and 9) were obtained in the same manner as in Example 6 mentioned previously, except that the types of base materials shown in Table 4 were used, and the tacky layers shown in Table 4 were formed.

### <Evaluation (2) of Peeling Resistance Using Skin Model>

The peeling resistance was evaluated for the produced drapes of Examples 6 and 7 and Comparative Examples 8 and 9 according to the same procedure as that of "Evaluation (1) of Peeling Resistance Using Skin Model" mentioned previously. The results are shown in Table 4.

**Table 4**

| | Base material | Tacky layer | | Performance evaluation (Peeling resistance) | |
|---|---|---|---|---|---|
| | | Material | Thickness (µm) | | |
| | | | | Individual | Average |
| Example 6 | 8 | Acrylic-based tackiness agent | 95 | 5 | 4.3 |
| | | | | 5 | |
| | | | | 3 | |
| Example 7 | 8 | Acrylic-based tackiness agent + silicone-based tackiness agent | 45+50 | 5 | 5 |
| | | | | 5 | |
| | | | | 5 | |
| Comparative Example 8 | 6 | Acrylic-based tackiness agent | 95 | 2 | 2.3 |
| | | | | 2 | |
| | | | | 3 | |
| Comparative Example 9 | 6 | Acrylic-based tackiness agent + silicone-based tackiness agent | 45+50 | 3 | 3.3 |
| | | | | 4 | |
| | | | | 3 | |

### INDUSTRIAL APPLICABILITY

The surgical drape of the present invention is particularly useful as a drape to be used for pasting it to a surgery site which is an uneven surface or a curved surface to incise the surgery site.

### REFERENCE SIGNS LIST

2: Tacky surface
5: Tacky layer
10: Surgical drape
15: Base material

## Claims

1. A surgical drape (10) comprising a tacky layer (5) to be incised or punctured with a surgical tool, the tacky layer (5) having a tacky surface (2) to be brought into contact with a region including a surgery site of a living body, **characterized in that** the surgical drape (10) has a Young's modulus in the range of 0.1 to 5.0 MPa or less, and **in that** the surgical drape (10) has a piercing strength in a thickness direction of 4.0 N or less.

2. The surgical drape (10) according to claim 1, further comprising a film-like base material (15) disposed by lamination on a surface on an opposite side of the tacky surface (2) of the tacky layer (5).

## Patentansprüche

1. Operationstuch (10) mit einer klebrigen Schicht (5), die mit einem chirurgischen Werkzeug eingeschnitten oder durchstochen werden soll, wobei die klebrige Schicht (5) eine klebrige Oberfläche (2) aufweist, die mit einem Bereich in Kontakt gebracht werden soll, der eine Operationsstelle eines lebenden Körpers umfasst, **dadurch gekennzeichnet, dass** das Operationstuch (10) einen Elastizitätsmodul im Bereich von 0,1 bis 5,0 MPa oder weniger aufweist, und dass das Operationstuch (10) eine Durchstoßfestigkeit in Dickenrichtung von 4,0 N oder weniger aufweist.

2. Das Operationstuch (10) gemäß Anspruch 1, das außerdem ein filmartiges Basismaterial (15) umfasst, das durch Laminieren auf einer Oberfläche auf einer der klebrigen Oberfläche (2) der klebrigen Schicht (5) gegenüberliegenden Seite angeordnet ist.

## Revendications

1. Champ opératoire (10) comprenant une couche adhésive (5) à inciser ou à perforer avec un outil chirurgical, la couche adhésive (5) ayant une surface adhésive (2) à mettre en contact avec une région comportant un site chirurgical d'un corps vivant, **caractérisé en ce que** le champ chirurgical (10) a un module de Young dans la plage de 0,1 à 5,0 MPa ou moins, et **en ce que** le champ opératoire (10) a une résistance au perçage dans une direction de l'épaisseur de 4,0 N ou moins.

2. Champ opératoire (10) selon la revendication 1, comprenant en outre un matériau de base (15) de type film disposé par stratification sur une surface d'un côté opposé de la surface adhésive (2) de la couche adhésive (5).
